# EUROPÄISCHE PATENTANMELDUNG

(11) **EP 4 310 030 A1**
(43) Veröffentlichungstag der Anmeldung: **24.01.2024**
(21) Anmeldenummer: 23183720.4
(22) Anmeldetag: 05.07.2023
(51) Int. Cl.: B65G 43/00, B65G 45/10, B65G 54/00, B65G 54/02, H02K 41/02, H02K 41/03

(54) **PLANARANTRIEBSVORRICHTUNG UND VERFAHREN ZUM BETRIEB EINER PLANARANTRIEBSVORRICHTUNG**

(30) Priorität: 20.07.2022 DE 102022118128
(71) Anmelder: Syntegon Technology GmbH, 71332 Waiblingen (DE)
(72) Erfinder: Schwarz, Sebastian, 91616 Neusitz (DE); Rauschnabel, Johannes, 74579 Fichtenau (DE); Kosian, Thomas, 91610 Insingen (DE)
(74) Vertreter: DREISS Patentanwälte PartG mbB

(57) **Zusammenfassung**

Die Erfindung betrifft eine Planarantriebsvorrichtung (10) mit wenigstens einem Mover (16) und einem Antriebstisch (12), wobei der Mover eine Außenhülle (40) aufweist, wobei der Antriebstisch eine ebene Antriebsfläche (14) aufweist und wobei der Mover elektromagnetisch mit der Antriebsfläche koppelbar und in einem Schwebezustand parallel zu der Antriebsfläche bewegbar ist, wobei der Antriebstisch einen Reinigungsbereich (30) mit einer Reinigungseinrichtung (32) zur Reinigung wenigstens eines Teils der Außenhülle des Movers aufweist, wobei der Mover durch elektromagnetischen Antrieb der Antriebsfläche in den Reinigungsbereich verbringbar ist, wobei die Reinigungseinrichtung mindestens eine auf oder oberhalb der Antriebsfläche angeordnete Zusatz-Reinigungseinheit (56) zur Reinigung einer Seitenfläche (58) und/oder einer Oberseite (60) des Movers aufweist.

## Beschreibung

Die Erfindung betrifft eine Planarantriebsvorrichtung mit den Merkmalen des Oberbegriffs des Patentanspruchs 1.

Aus der US 2022/0032477 A2 und der DE 10 2019 117 431 A1 sind Planarantriebsvorrichtungen mit einem Antriebstisch und elektromagnetisch gekoppelten Movern bekannt. Derartige Vorrichtungen können beispielsweise für den Transport unterschiedlicher Transportgüter und/oder zur Anordnung und zum Betrieb von Arbeitswerkzeugen genutzt werden.

Während des Betriebs solcher Planarantriebsvorrichtungen kann es regelmäßig zu Verunreinigungen der Außenhülle der Mover kommen. Beispielsweise können Stoffe aus der direkten Umgebung der Vorrichtung aufgenommen werden, und/oder verschüttete Medien aus einem Transportgut bleiben an der Außenhülle der Mover haften.

Für einen störungsfreien Betriebsablauf ist es gewünscht, die Verunreinigungen von der Außenhülle der Mover zu entfernen.

Hiervon ausgehend liegt der Erfindung die Aufgabe zugrunde, eine Vorrichtung sowie ein Verfahren anzugeben, welche eine einfache Reinigung der Außenhülle der Mover, insbesondere während eines laufenden Betriebs der Vorrichtung, ermöglichen.

Diese Aufgabe wird erfindungsgemäß durch eine Planarantriebsvorrichtung mit den Merkmalen des Patentanspruchs 1 gelöst.

Durch die Anordnung der Reinigungseinrichtung an dem Antriebstisch ist es möglich, eine immanente Funktion der Vorrichtung - nämlich die Bewegbarkeit des Movers - zu nutzen, um den Mover in den Reinigungsbereich und damit in eine direkte räumliche Nähe zu der Reinigungseinrichtung zu verbringen.

Auf diese Weise ist es möglich, die Reinigung der Außenhülle des Movers in den Betriebsablauf zu integrieren. Beispielsweise kann der Mover bei der Bewegung zwischen Abgabe- und Aufnahmeort eines Transportguts in den Reinigungsbereich verbracht und dort gereinigt werden, wodurch der zeitliche Aufwand für die Reinigung minimiert wird und insbesondere auf einen manuellen Eingriff verzichtet werden kann.

Die Vorrichtung eignet sich insbesondere für einen Einsatz als Teil einer pharmazeutischen Anlage. Dort werden besonders hohe Ansprüche an die Sauberkeit und Sterilität der einzelnen Komponenten und insbesondere der Außenhülle der Mover gestellt.

Bei einer bevorzugten Ausführungsform weist die Reinigungseinrichtung eine Reinigungseinheit auf, welche mit einer der Antriebsfläche zugewandten Unterseite des Movers zusammenwirkt. Aufgrund eines nur kleinen Abstands zwischen dem Mover und der Antriebsfläche ist die Unterseite des Movers besonders schwer für die Reinigung zugänglich. Da außerdem die elektromagnetischen Kräfte zwischen der Antriebsfläche und der Unterseite des Movers wirksam sind, sammeln sich dort insbesondere magnetisch wirksame Verunreinigungen an. Wirkt die Reinigungseinheit mit der Unterseite des Movers zusammen, können dort gezielt Verunreinigungen entfernt werden.

Besonders bevorzugt weist die Antriebsfläche eine Mehrzahl von kachelförmigen Antriebsabschnitten auf, wobei eine Reinigungseinheit der Reinigungseinrichtung in einem zwischen benachbarten Antriebsabschnitten ausgebildeten Spalt angeordnet ist. Die Anordnung der Reinigungseinrichtung in einem Spalt zwischen benachbarten Antriebsabschnitten ermöglicht es, die Bewegungsfreiheit des Movers auf der gesamten Antriebsfläche zu erhalten und dennoch eine Möglichkeit zur Reinigung der Außenhülle des Movers bereitzustellen. Eine bevorzugte Mindest-Spaltbreite beträgt 3 mm. Eine bevorzugte Maximal-Spaltbreite beträgt 60 mm.

Insbesondere ist es bevorzugt, dass eine Spaltbreite nicht größer ist als 30% einer Länge oder einer Breite eines Moverkörpers (jeweils parallel zu der Antriebsfläche gemessen).

Ferner ist es bevorzugt, dass der Reinigungsbereich durch die Reinigungseinrichtung zumindest in Richtung der Antriebsfläche begrenzt ist. Auf diese Weise ist gewährleistet, dass die Reinigungseinrichtung möglichst nahe an dem Reinigungsbereich und damit nahe an der Außenhülle des Movers angeordnet ist und zumindest die Unterseite des Movers besonders effektiv gereinigt werden kann.

Erfindungsgemäß weist die Reinigungseinrichtung mindestens eine auf oder oberhalb der Antriebsfläche angeordnete Zusatz-Reinigungseinheit zur Reinigung einer Seitenfläche und/oder einer Oberseite des Movers auf. Dies ermöglicht die Reinigung weiterer Teilbereiche der Außenhülle oder der gesamten Außenhülle des Movers in dem Reinigungsbereich. Die mindestens eine Zusatz-Reinigungseinheit kann dabei derart angeordnet sein, dass eine gleichzeitige Reinigung der Unterseite einerseits und der Seitenfläche und/oder Oberseite des Movers andererseits möglich ist.

Weiter ist es bevorzugt, dass die Reinigungseinrichtung mindestens ein Sprühelement zum Versprühen eines Reinigungsmediums und/oder eine UV-Quelle umfasst. Das Versprühen erlaubt eine gezielte und besonders gleichmäßige Aufbringung des Reinigungsmediums auf der Außenhülle des Movers. Die Anzahl der Sprühelemente kann dabei auf die Breite des Movers abgestimmt sein, um ein besonders effizientes Sprühbild zu erzeugen und den Verbrauch des Reinigungsmediums zu minimieren. Der Einsatz der UV-Quelle stellt eine besonders effektive Möglichkeit gegenüber biologischen Verunreinigungen dar und kann in der Reinigungseinrichtung auf einfache Art und Weise mit den Sprühelementen kombiniert werden.

Des Weiteren ist es bevorzugt, dass die Reinigungseinrichtung mechanische Reinigungselemente umfasst. Mechanische Reinigungselemente, bspw. Bürsten und/oder Schwämme, ermöglichen die Entfernung von besonders hartnäckigen Verunreinigungen. Vorzugsweise wird der Mover in Kontakt mit den mechanischen Reinigungselementen gebracht und relativ zu den stationären Reinigungselementen bewegt. Es ist auch möglich, dass die Reinigungseinrichtung eine Kombination aus mechanischen Reinigungselementen und Sprühelementen und/oder der UV-Quelle aufweist.

Insbesondere ist es bevorzugt, dass der Reinigungsbereich eine Abflusseinrichtung und/oder eine Absaugeinrichtung umfasst. Hierdurch kann ein Überschuss des Reinigungsmediums und insbesondere ein Rückfluss des Reinigungsmedium nach erfolgter Reinigung auf einfache Art und Weise von dem Antriebstisch entfernt und gesammelt werden.

Ferner ist es bevorzugt, dass die Abflusseinrichtung und/oder die Absaugeinrichtung in die Reinigungseinrichtung integriert ist. Dies stellt eine einfache Möglichkeit dar, die Aufbringung und die Entfernung des Reinigungsmediums in dem Reinigungsraum ohne zusätzliche bauliche Veränderungen an dem Antriebstisch zu realisieren.

Zwecks Reduzierung eines zeitlichen Aufwands ist es besonders bevorzugt, dass in dem Reinigungsbereich gleichzeitig eine Mehrzahl von Movern anordenbar und reinigbar ist.

Die eingangs genannte Aufgabe wird auch durch ein Verfahren zum Betrieb einer Planarantriebsvorrichtung gelöst, welches die Merkmale des nebengeordneten Verfahrensanspruchs umfasst.

Das erfindungsgemäße Verfahren sieht vor, dass der Mover in dem Reinigungsbereich angeordnet und dass zumindest ein Teil der Außenhülle des Movers mittels der Reinigungseinrichtung gereinigt wird. Dabei ist es möglich, dass der Mover in dem Reinigungsbereich stillsteht oder derart angetrieben wird, dass sich der Mover in dem Reinigungsbereich mit einer konstanten Geschwindigkeit bewegt.

Das Verfahren ermöglicht es, die Reinigung der Außenhülle des Movers in den Betriebsablauf zu integrieren und den Zeitaufwand für die Reinigung zu minimieren.

Gemäß einem bevorzugten Verfahren führt der Mover in dem Reinigungsbereich eine Rotationsbewegung um seine Hochachse aus. Dadurch wird eine gleichmäßige Aufbringung des Reinigungsmediums und/oder UV-Lichts auf der Außenhülle des Movers erreicht.

Ferner ist es möglich, dass der Mover in dem Reinigungsbereich oszillierende Bewegungen entlang und/oder um seine Querachse und/oder Längsachse und/oder Hochachse ausführt. Die oszillierende Bewegung unterstützt eine Verteilung eines Reinigungsmedium auf der Außenhülle des Movers.

Weitere Merkmale und Vorteile sind Gegenstand der nachfolgenden Beschreibung und der zeichnerischen Darstellung von Ausführungsformen.

In der Zeichnung zeigt
- Fig. 1: eine Draufsicht einer Ausführungsform einer Planarantriebsvorrichtung mit einem Mover und einer Antriebsfläche mit einem Reinigungsbereich;
- Fig. 2: eine Vorderansicht der Planarantriebsvorrichtung längs einer in Fig. 1 mit II - II bezeichneten Schnittebene;
- Fig. 3: eine Vorderansicht der Planarantriebsvorrichtung längs einer in Fig. 1 mit III - III bezeichneten Schnittebene, mit dem Reinigungsbereich in vergrößerter Darstellung;
- Fig. 4: eine Seitenansicht des Reinigungsbereichs längs einer in Fig. 3 mit IV - IV bezeichneten Schnittebene;
- Fig. 5 und 6: der Fig. 4 entsprechende Ansichten der Planarantriebsvorrichtung mit Zusatz-Reinigungseinheiten zur Reinigung von Seitenflächen und/oder Oberseiten des Movers;
- Fig. 7: eine der Fig. 4 entsprechende Ansicht einer weiteren Ausführungsform einer Planarantriebsvorrichtung.

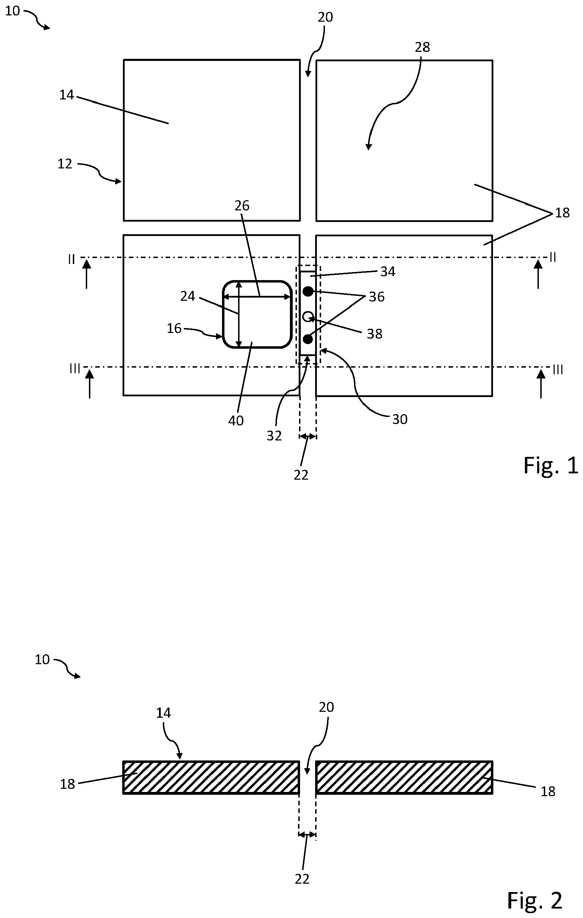

Eine Planarantriebsvorrichtung ist in der Zeichnung insgesamt mit dem Bezugszeichen 10 bezeichnet. Die Planarantriebsvorrichtung 10 umfasst einen stationären Antriebstisch 12 mit einer ebenen Antriebsfläche 14 und einen Mover 16, welcher elektromagnetisch mit der Antriebsfläche 14 gekoppelt ist. Der Mover 16 ist in einem Schwebezustand auf der Antriebsfläche 14 antreibbar und frei positionierbar, vergleiche Fig. 1.

Der Antriebstisch 12 umfasst eine Mehrzahl kachelförmiger Antriebsabschnitte 18. Die Antriebsabschnitte 18 sind derart angeordnet, dass benachbarte Antriebsabschnitte 18 durch einen Spalt 20 mit einer festen Spaltbreite 22 voneinander getrennt sind, vergleiche Fig. 1 und 2.

Der Mover 16 ist über den Spalt 20 hinweg antreibbar, wobei die Spaltbreite 22 vorzugsweise deutlich kleiner ist als eine Breite 24 oder Länge 26 des Movers 16. Beispielsweise ist die Breite 22 des Spalts 20 nicht größer als 30% der Breite 24 oder der Länge 26 des Movers 16.

Die Antriebsfläche 14 weist einen Arbeitsbereich 28 auf. In dem Arbeitsbereich 28 kann der Mover 16 unterschiedliche Arbeitsfunktionen realisieren, bspw. ein Transportgut transportieren und/oder mit Arbeitswerkzeugen (nicht dargestellt) interagieren.

Die Antriebsfläche 14 weist außerdem einen Reinigungsbereich 30 auf. Der Reinigungsbereich 30 erstreckt sich beispielsweise zwischen zwei benachbarten Antriebsabschnitten 18, insbesondere entlang einer Teillänge eines Spalts 20 der Arbeitsfläche 14. In dem Reinigungsbereich 30 ist eine Reinigungseinrichtung 32 mit einer Reinigungseinheit 34 angeordnet, welche den Reinigungsbereich 30 in Richtung der Antriebsfläche 14 begrenzt.

Die Reinigungseinrichtung 32 weist senkrecht zu der Antriebsfläche 14 orientierte Sprühelemente 36 zum Versprühen eines Reinigungsmediums auf, ferner optional eine Abflusseinrichtung 38. Es ist ebenso denkbar, an der Reinigungseinheit 34 eine oder mehrere UV-Quellen oder eine Kombination aus Sprühelementen 36 und UV-Quellen anzuordnen.

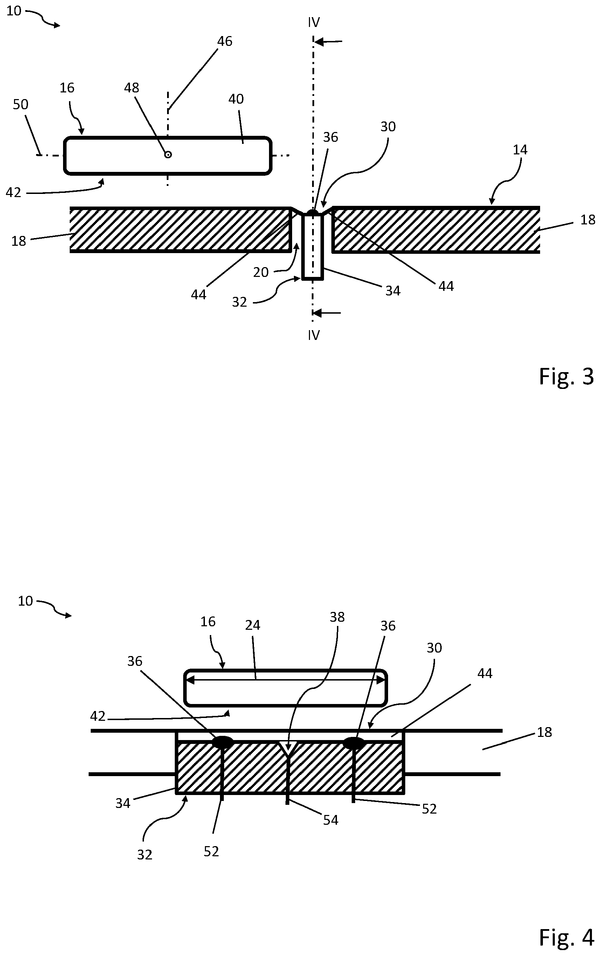

Die Reinigungseinrichtung 32 dient der Reinigung wenigstens eines Teils einer Außenhülle 40 des Movers 16, insbesondere einer der Antriebsfläche zugewandten Unterseite 42 des Movers 16, vergleiche Fig. 3.

Die Reinigungseinheit 34 ist bezogen auf die Antriebsfläche 14 benachbarter Antriebsabschnitte 18 rückversetzt angeordnet, wobei der Versatz durch Verbindungsprofile 44 ausgeglichen ist, deren Oberseiten relativ zu der Antriebsfläche 14 geneigt sind.

Zur Reinigung der Unterseite 42 wird der Mover 16 durch den elektromagnetischen Antrieb der Antriebsfläche 14 von dem Arbeitsbereich 28 in den Reinigungsbereich 30 verbracht, und die Unterseite 42 wird durch die Sprühelemente 36 mit dem Reinigungsmedium beaufschlagt. Dabei kann der Mover 16 in dem Reinigungsbereich 30 stillstehen oder sich mit einer konstanten Geschwindigkeit durch den Reinigungsbereich 30 hindurch bewegen.

Für eine besonders gleichmäßige Verteilung des Reinigungsmediums ist es möglich, dass der Mover 16 - dem Stillstand oder der konstanten Bewegung überlagert - eine Rotationsbewegung um seine Hochachse 46 und/oder oszillierende Bewegungen um und/oder entlang seine Querachse 48 und/oder Längsachse 50 und/oder Hochachse 46 ausführt.

Um ein Reinigungsmedium möglichst effizient zu nutzen, ist es bevorzugt, dass die Anzahl der Sprühelemente 36 sowie deren Positionierung und Verteilung in der Reinigungseinheit 34 auf die Breite 24 und/oder Länge 26 des Movers 16 abgestimmt, vergleiche Fig. 4, welche eine Schnittdarstellung der Vorrichtung 10 entlang der in Fig. 3 mit IV-IV markierten Schnittebene zeigt. Die Sprühelemente 36 sind über Leitungen 52 mit einem nicht dargestellten und bevorzugt in oder an dem Antriebstisch 12 angeordneten Reinigungsmediumsreservoir sowie einem Pumpensystem verbunden und können vorzugsweise gruppiert oder individuell betätigt werden.

Die Abflusseinrichtung 38 ist beispielsweise als eine mittig angeordnete, trichterförmige Vertiefung in der Reinigungseinheit 34 ausgebildet. An ihrem verjüngten Ende ist die Vertiefung mit einer innerhalb der Reinigungseinheit 34 verlaufenden Abflussleitung 54 verbunden, wodurch überschüssiges und zurücklaufendes, ggf. mit Schmutz des Movers 16 versehenes Reinigungsmedium von der Antriebsfläche 14 abgeführt werden kann.

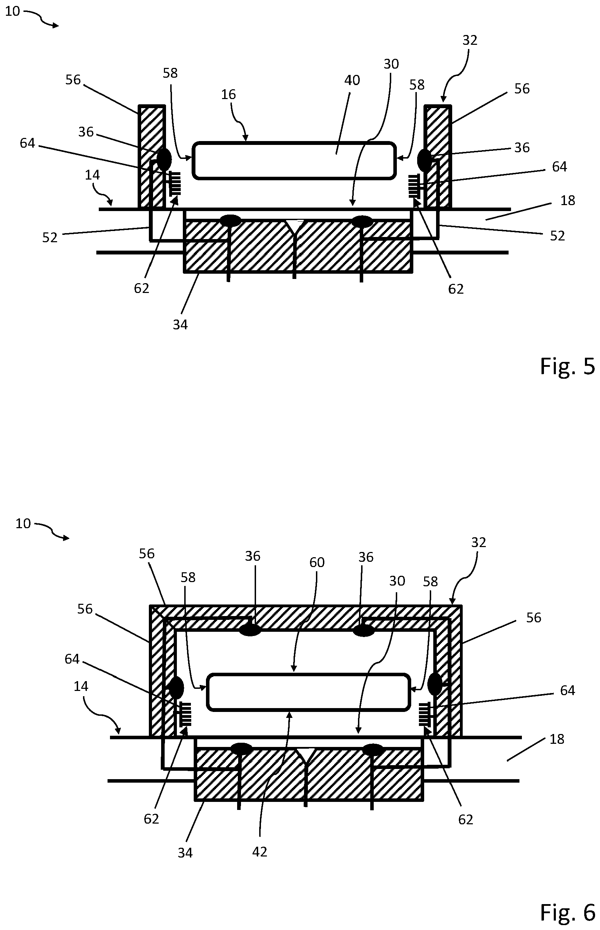

Es ist möglich, die Reinigungseinrichtung 32 durch eine oder mehrere Zusatz-Reinigungseinheiten 56 zu erweitern, vergleiche Fig. 5 und 6. Die Zusatz-Reinigungseinheiten 56 sind auf oder oberhalb der Antriebsfläche 14 angeordnet und bilden ihrerseits eine Begrenzung des Reinigungsbereichs 30. Die Zusatz-Reinigungseinheiten 56 sind insbesondere derart angeordnet, dass eine gleichzeitige Reinigung der Unterseite 42 sowie von Seitenflächen 58 und/oder einer Oberseite 60 des Movers 16 möglich ist.

Zur Beaufschlagung der Außenhülle 40 des Movers 16 mit dem Reinigungsmedium weisen die Zusatz-Reinigungseinheiten 56 ebenfalls Sprühelemente 36 und Leitungen 52 auf.

Zusätzlich zu den Sprühelementen 36 können die Reinigungseinheit 34 und/oder die Zusatz-Reinigungseinheiten 56 mechanische Reinigungselemente 62, bspw. Bürsten 64 umfassen. Zur Reinigung wird der Mover 16 während und/oder nach der Beaufschlagung mit dem Reinigungsmedium mit den mechanischen Reinigungselementen 62 in Kontakt gebracht und relativ zu den mechanischen Reinigungselementen 62 bewegt.

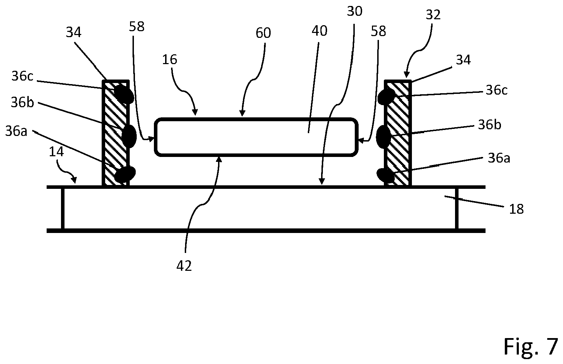

In Fig. 7 ist eine weitere Ausführungsform der Planarantriebsvorrichtung 10 in einer der Fig. 4 bis 6 entsprechenden Schnittdarstellung gezeigt. Abweichend von der vorstehend beschriebenen Ausführungsform ist die Reinigungseinrichtung 32 nicht in einem Spalt zwischen benachbarten Antriebsabschnitten 18 angeordnet, sondern auf oder oberhalb der Antriebsfläche 14.

Die Reinigungseinrichtung 32 umfasst Reinigungseinheiten 34 mit Sprühelementen 36a bis 36c, welche unterschiedliche Sprührichtungen aufweisen. Die Sprühelemente 36a bis 36c sind mit unterschiedlichen Abständen zu der Antriebsfläche 14 beabstandet angeordnet und weisen relativ zueinander vorzugsweise identische Abstände auf.

Die Reinigungseinheiten 34 weisen benachbart zu der Antriebsfläche 14 angeordnete Sprühelemente 36a auf, welche das Reinigungsmedium von der Antriebsfläche 14 abgewandt in Richtung auf die Unterseite 42 des in dem Reinigungsbereich 30 angeordneten Movers 16 sprühen.

Die Reinigungseinheiten 34 weisen bezogen auf die Sprühelemente 36a weiter zu der Antriebsfläche 14 beabstandete Sprühelemente 36b auf, welche das Reinigungsmedium in einer zu der Antriebsfläche 14 parallelen Richtung auf die Seitenflächen 58 des in dem Reinigungsbereich 30 angeordneten Movers 16 sprühen.

Die Reinigungseinheiten 34 weisen bezogen auf die Sprühelemente 36b weiter zu der Antriebsfläche 14 beabstandete Sprühelemente 36c auf, welche das Reinigungsmedium in Richtung auf die Antriebsfläche auf die Oberseite 60 des in dem Reinigungsbereich 30 angeordneten Movers 16 sprühen.

Wird der Mover 16 in den Reinigungsbereich 30 verbracht, ist es auch mit der Anordnung gemäß Fig. 7 möglich, die gesamte Außenhülle 40 des Movers 16 zu reinigen, wobei unterhalb und/oder oberhalb der Antriebsfläche 14 keine Reinigungseinheiten 34 angeordnet sein müssen (also abweichend von den Anordnungen gemäß Fig. 5 und 6).

## Patentansprüche

1. Planarantriebsvorrichtung (10) mit wenigstens einem Mover (16) und einem Antriebstisch (12), wobei der Mover (16) eine Außenhülle (40) aufweist, wobei der Antriebstisch (12) eine ebene Antriebsfläche (14) aufweist und wobei der Mover (16) elektromagnetisch mit der Antriebsfläche (14) koppelbar und in einem Schwebezustand parallel zu der Antriebsfläche (14) bewegbar ist, wobei der Antriebstisch (12) einen Reinigungsbereich (30) mit einer Reinigungseinrichtung (32) zur Reinigung wenigstens eines Teils der Außenhülle (40) des Movers (16) aufweist, wobei der Mover (16) durch elektromagnetischen Antrieb der Antriebsfläche (14) in den Reinigungsbereich (30) verbringbar ist, **dadurch gekennzeichnet, dass** die Reinigungseinrichtung (32) mindestens eine auf oder oberhalb der Antriebsfläche (14) angeordnete Zusatz-Reinigungseinheit (56) zur Reinigung einer Seitenfläche (58) und/oder einer Oberseite (60) des Movers (16) aufweist.

2. Planarantriebsvorrichtung (10) nach Anspruch 1, **dadurch gekennzeichnet, dass** die Reinigungseinrichtung (32) eine Reinigungseinheit (34) aufweist, welche mit einer der Antriebsfläche (14) zugewandten Unterseite (42) des Movers (16) zusammenwirkt.

3. Planarantriebsvorrichtung (10) nach einem der vorstehenden Ansprüche, **dadurch gekennzeichnet, dass** die Antriebsfläche (14) eine Mehrzahl von kachelförmigen Antriebsabschnitten (18) aufweist und dass eine Reinigungseinheit (34) der Reinigungseinrichtung (32) in einem zwischen benachbarten Antriebsabschnitten (18) ausgebildeten Spalt (20) angeordnet ist.

4. Planarantriebsvorrichtung (10) nach einem der vorstehenden Ansprüche, **dadurch gekennzeichnet, dass** der Reinigungsbereich (30) durch die Reinigungseinrichtung (32) zumindest in Richtung der Antriebsfläche (14) begrenzt ist.

5. Planarantriebsvorrichtung (10) nach einem der vorstehenden Ansprüche, **dadurch gekennzeichnet, dass** die Reinigungseinrichtung (32) mindestens ein Sprühelement (36) zum Versprühen eines Reinigungsmediums und/oder eine UV-Quelle umfasst.

6. Planarantriebsvorrichtung (10) nach einem der vorstehenden Ansprüche, **dadurch gekennzeichnet, dass** die Reinigungseinrichtung (32) mechanische Reinigungselemente (62) umfasst.

7. Planarantriebsvorrichtung (10) nach einem der vorstehenden Ansprüche, **dadurch gekennzeichnet, dass** der Reinigungsbereich (30) eine Abflusseinrichtung (38) und/oder eine Absaugeinrichtung umfasst.

8. Planarantriebsvorrichtung nach Anspruch 7, **dadurch gekennzeichnet, dass** die Abflusseinrichtung (38) und/oder Absaugeinrichtung in die Reinigungseinrichtung (32) integriert ist.

9. Planarantriebsvorrichtung (10) nach einem der vorstehenden Ansprüche, **dadurch gekennzeichnet, dass** in dem Reinigungsbereich (30) gleichzeitig eine Mehrzahl von Movern (16) anordenbar und reinigbar ist.

10. Verfahren zum Betrieb einer Planarantriebsvorrichtung (10) nach einem der vorstehenden Ansprüche, wobei der Mover (16) in dem Reinigungsbereich (30) angeordnet wird und wobei zumindest ein Teil der Außenhülle (40) des Movers (16) mittels der Reinigungseinrichtung (32) gereinigt wird, wobei während der Reinigung der Mover (16) in dem Reinigungsbereich (30) stillsteht oder derart angetrieben wird, dass sich der Mover (16) in dem Reinigungsbereich (30) mit einer konstanten Geschwindigkeit bewegt.

11. Verfahren nach Anspruch 10, wobei der Mover (16) in dem Reinigungsbereich (30) eine Rotationsbewegung um seine Hochachse (46) ausführt.

12. Verfahren nach Anspruch 10 oder 11, wobei der Mover (16) in dem Reinigungsbereich (30) oszillierende Bewegungen entlang und/oder um seine Querachse (48) und/oder Längsachse (50) und/oder Hochachse (46) ausführt.
